# EUROPEAN PATENT SPECIFICATION

(11) **EP 2 105 741 B1**
(45) Date of publication and mention of the grant of the patent: **06.07.2011**
(21) Application number: 09158701.4
(22) Date of filing: 02.03.2004
(51) Int. Cl.: G01N 33/58, G01N 33/543

(54) **Use of a virus expressing a binding moiety to measure analytes in a sample**
Verwendung eines Bindungsteil exprimierenden Virus zur Messung von Analyten in einer Probe
Utilisation d'un virus exprimant un groupe caractéristique de liaison pour mesurer des analytes dans un échantillon

(30) Priority: 04.03.2003 GB 0304832
(43) Date of publication of application: 30.09.2009
(62) Divisional of application: 07011811.2
(73) Proprietor: The Secretary of State for Defence, Salisbury, Wiltshire SP4 0JQ (GB)
(72) Inventor: Squirrell, David James, Salisbury, Wiltshire SP4 0JQ (GB); Lee, Martin Alan, Salisbury, WIltshire SP4 0JQ (GB); Mayers, Carl Nicholas, Salisbury, Wiltshire SP4 0JQ (GB)
(74) Representative: Turner, Rhiannon Rosalind

(56) References cited:
- EP-A- 0 402 997
- EP-A- 1 122 542
- US-A- 5 403 484
- US-A- 5 922 537
- US-B1- 6 265 169
- US-B1- 6 365 368
- HUDSON L. ET AL.: "PRACTICAL IMMUNOLOGY" 1989, BLACKWELL SCIENTIFIC , OXFORD, UK , XP002536799 ISBN: 0632014911 * page 351 *
- KLEIN DIETER: "Quantification using real-time PCR technology: Applications and limitations" TRENDS IN MOLECULAR MEDICINE, vol. 8, no. 6, June 2002 (2002-06), pages 257-260, XP002461653 ISSN: 1471-4914
- EDWARDS M C ET AL: "Multiplex PCR: Advantages, development, and applications" PCR METHODS AND APPLICATIONS 1994 UNITED STATES, vol. 3, no. 4, 1994, pages S65-S75, XP002461654 ISSN: 1054-9803
- EKINS R ET AL: "IMMUNOASSAY AND OTHER LIGAND ASSAYS: PRESENT STATUS AND FUTURE TRENDS" INTERNATIONAL FEDERATION OF CLINICAL CHEMISTRY. JOURNAL, vol. 9, no. 3, September 1997 (1997-09), pages 100,102-109, XP001153007

## Description

The present invention provides a novel assay method.

Immuno-PCR, which dates from 1992, uses nucleic acid tagged antibodies to provide a very sensitive assay endpoint. Generally the antibody is labelled with streptavidin and the nucleic acid through the streptavidin to the antibody. The biotinylated DNA is usually added at the end of the immunoassay.

Viruses may comprise essentially DNA or RNA, and attack host cells, integrating their nucleic acids into the host system. In structural terms, viruses generally express proteins which form a "coat" around the nucleic acids.

Phage display is a technique that was developed to allow proteins such as antibodies to be selected and produced *in vitro.* Phage libraries are made that contain a very high number of different proteins, such as scFv's (single chain variable fragments from antibodies). The phage has the DNA for the protein scFv's inserted into its genome and it expresses it as a fusion protein to the coat proteins, generally attached at its head.

The best protein such as scFv for a particular purpose is selected from the library mixture by "panning" for the phage that binds to the analyte of interest under strict selection conditions. The phage can then be multiplied by growth in its host bacterium and the DNA can be cut out and inserted into an expression vector. Binding protein can then be produced either as scFv or incorporated back into an antibody framework to make, for example, humanised antibodies for therapeutic use.

The phage display technique is thus used as an intermediate technique for *in vitro* antibody production. However, the phages themselves have never been proposed for use as assay reagents previously. US6265169 describes a method for using filamentous bacteriophages to detect the presence of molecules of interest in biological samples.

Thus according to the present invention there is provided a method of detecting an analyte in a liquid sample, said method comprising passing said sample through a filter so that analyte is retained on the filter, washing the filter, passing a suspension of filamentous phage expressing an scFV specific for an assay target selected from the analyte or an analogue thereof, or a binding moiety for the analyte, through the filter, washing the filter, detecting any phage which has bound to the assay target on the filter by detecting the presence or absence of phage DNA on the filter using a polymerase chain reaction, and relating that to the presence or absence of analyte in the sample.

The filter may be a 0.2 micron filter or a 0.45 micron filter.

The analyte may comprise bacterial spores.

The suspension of filamentous phage may comprise a multi-specificity mixture of viruses, wherein detection of multiple nucleic acid sequences, each characteristic of individual viruses, may be carried out to detect the presence or absence of phage.

The amplification reaction may be carried out in the presence of a DNA binding reagent, or a primer or probe which is labelled with a fluorescent label.

The amount of filamentous phage detected may be quantified and related to the amount of analyte in the sample.

The phage may be detected by detecting a nucleic acid sequence which is characteristic of a particular phage used in the method. The phage may be a recombinant virus which has been transformed with a marker sequence, this sequence being the sequence which is detected. Alternatively, sequences characteristic of more than one virus may be detected.

The expression "binding moiety" relates to any moiety which will bind to a target, especially a polypeptide or protein, which may be for example an analyte polypeptide or protein, but may also be another polypeptide or protein, which is utilised in an immunoassay as part of the detection system.

The nucleic acid of the virus acts as a label, which may be detected using any of the known nucleic acid detection methods, in particular by using amplification reactions such as the polymerase chain reaction. However, the advantage of using a virus as compared to any other labelling technique is that a wide variety of binding moieties can be included relatively simply using techniques known for example for the production of recombinant viruses, such as phage display libraries, and without the need for additional binding steps.

Analytes are generally proteins or polypeptides. Typical examples will be polypeptides or proteins that are associated with or part of a pathogenic organism such as a virus, bacteria or bacterial spore such as anthrax or anthrax spores, or a protein which is indicative of a particular disease state or of exposure to a particular disease, such as an immunoglobulin for instance an antibody.

The nucleic acid sequence of the virus is detected using a polymerase chain reaction (PCR). Reagents, including primers, polymerase, nucleotides and buffers as are well known, are added and then subjected to thermal cycling as is conventional, in order to amplify any target nucleic acid sequence present.

Preferably the polymerase chain reaction is carried out in such a way that the amplification product generates a detectable signal, and in particular a visible signal, for example a fluorescent signal, as it progresses. Many assay formats that produce such signals are known in the art. They may utilise reagents such as DNA binding agents such as intercalating dyes which emit radiation and particularly fluorescent radiation at greater intensity when they are intercalated into double stranded DNA, as well as probes and primers which include fluorescent labels, arranged to undergo fluorescent energy transfer (FET) and particularly fluorescent resonant energy transfer (FRET).

There are two commonly used types of FET or FRET probes, those using hydrolysis of nucleic acid probes to separate donor from acceptor, and those using hybridisation to alter the spatial relationship of donor and acceptor molecules.

Hydrolysis probes are commercially available as TaqMan™ probes. These consist of DNA oligonucleotides that are labelled with donor and acceptor molecules. The probes are designed to bind to a specific region on one strand of a PCR product. Following annealing of the PCR primer to this strand, *Taq* enzyme extends the DNA with 5' to 3' polymerase activity. *Taq* enzyme also exhibits 5' to 3' exonuclease activity. TaqMan™ probes are protected at the 3' end by phosphorylation to prevent them from priming *Taq* extension. If the TaqMan™ probe is hybridised to the product strand, an extending *Taq* molecule may also hydro lyse the probe, liberating the donor from acceptor as the basis of detection. The signal in this instance is cumulative, the concentration of free donor and acceptor molecules increasing with each cycle of the amplification reaction.

Hybridisation probes are available in a number of forms. Molecular beacons are oligonucleotides that have complementary 5' and 3' sequences such that they form hairpin loops. Terminal fluorescent labels are in close proximity for FRET to occur when the hairpin structure is formed. Following hybridisation of molecular beacons to a complementary sequence the fluorescent labels are separated, so FRET does not occur, and this forms the basis of detection.

Pairs of labelled oligonucleotides may also be used. These hybridise in close proximity on a PCR product strand-bringing donor and acceptor molecules together so that FRET can occur. Enhanced FRET is the basis of detection. Variants of this type include using a labelled amplification primer with a single adjacent probe.

Other methods for detecting amplification reactions as they occur are known however, and any of these may be used. Particular examples of such methods are described for example in WO 99/28500, British Patent No. 2,338,301, WO 99/28501 and WO 99/42611.

WO 99/28500 describes a very successful assay for detecting the presence of a target nucleic acid sequence in a sample. In this method, a DNA duplex binding agent and a probe specific for said target sequence, is added to the sample. The probe comprises a reactive molecule able to absorb fluorescence from or donate fluorescent energy to said DNA duplex binding agent. This mixture is then subjected to an amplification reaction in which target nucleic acid is amplified, and conditions are induced either during or after the amplification process in which the probe hybridises to the target sequence. Fluorescence from said sample is monitored.

An alternative form of this assay, which utilises a DNA duplex binding agent which can absorb fluorescent energy from the fluorescent label on the probe but which does not emit visible light, is described in co-pending British Patent Application No. 223563.8. Any of these assays may be used in the context of the assay method of the invention in order to detect the target nucleic acid sequence.

Many of these assays can be carried out in a quantitative manner as is well known in the art, for example by monitoring the signal from the amplification mixture at least once during each cycle of the amplification reaction. By carrying out the reaction in this way, the amount of virus present on the filter may be determined, and this may be related to the amount of analyte present in the original sample.

If desired, specific marker sequences may be included in the virus in addition to those coding for the binding moiety. They may be introduced into the virus at the same time as the binding moiety, for example fused to the sequence encoding the binding moiety, or may be added in a separate transformation operation.

### Figures

Figure 1 illustrates diagrammatically, a sandwich assay ;
Figure 2 illustrates diagrammatically a competitive assay ;
Figure 3 is a graph of fluorescence -d (F1) / dt versus temperature when carrying out a PCR reaction of the TASMAN® type;
Figure 4 is a graph of fluorescence (Fl) against cycle number of series of samples at different dilutions using the assay ; and
Figure 5 shows the results of an assay described hereinafter for *B. cereus* spores including a PCR for detecting phage DNA.

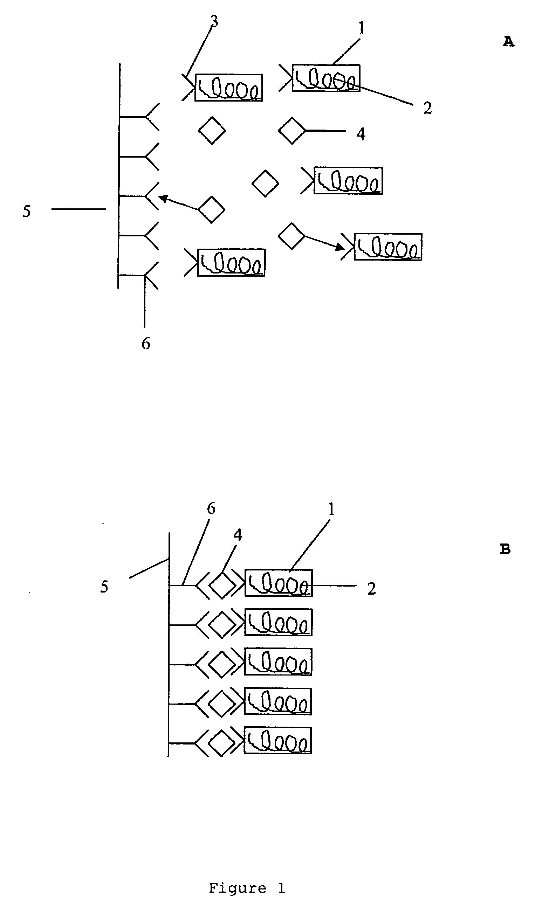

In the sandwich assay illustrated in Figure 1A, a phage (1) is used, which comprises an outer coat, enclosing phage DNA (2). A binding partner (3) such as an scFv is expressed by the phage and displayed on the surface at the head of the phage. It is added as a reagent to an assay reaction mixture which may contain analyte (4), and is in contact with a surface, such as a bead or well (5) on which an antibody (6) which is also specific for the analyte (4) is immobilised.

On incubation (B), the phage (1) and analyte (4) forms a complex which is retained on the surface (5), by the binding of the analyte to the antibody (6). Thereafter, the surface (5) is removed from the remainder of the sample and washed. However, some phage is retained on the surface, where it may be detected.

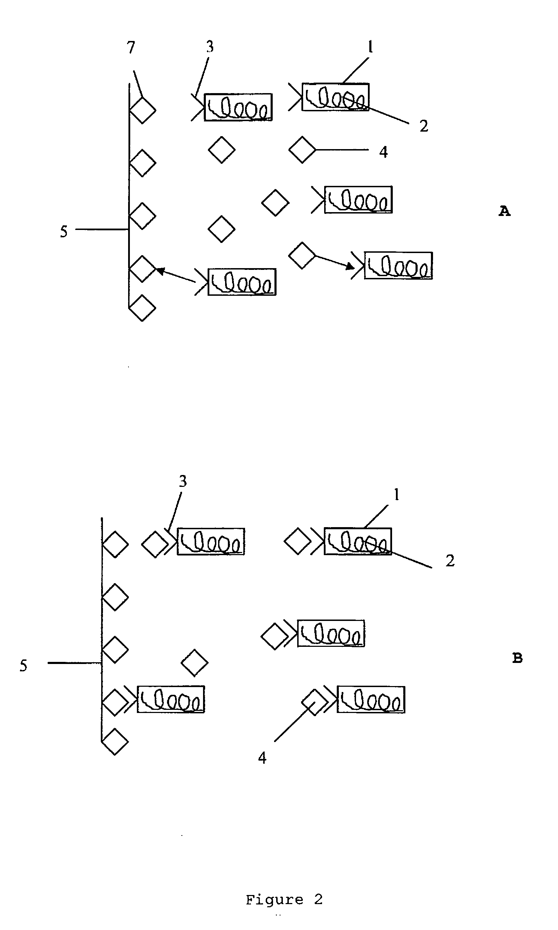

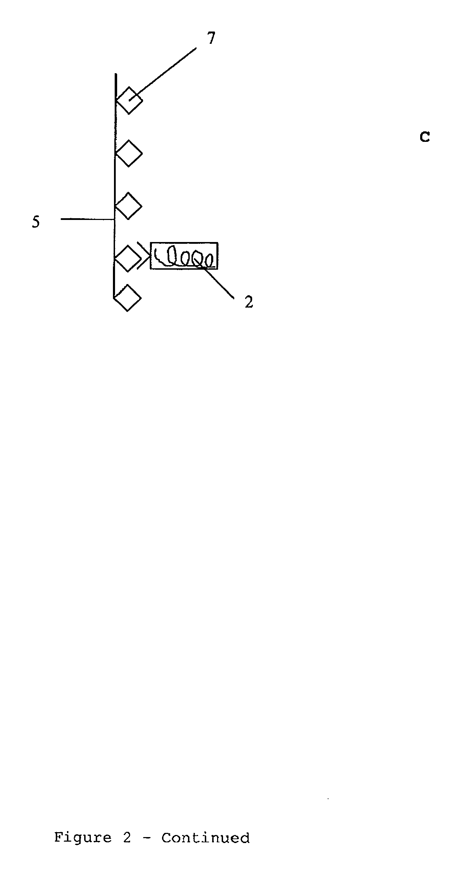

In Figure 2A, an analyte or an analogue thereof (7) capable of binding to the binding partner (3) of the phage (1) is immobilised on the surface (5). A sample under test to which the phage (1) has been added is incubated in the presence of this surface. Analyte (4) in the sample will bind to the binding partner (3) of the phage (1). Any phage which has undergone such binding is unable to bind to the immobilised analyte analogue (7) (B), and therefore will be washed away with the sample during a subsequent separation step. Detection of phage DNA (2) on the surface (5) following such a washing step will reveal lower levels than would otherwise be expected if no analyte were present.

### Example 1

### Demonstration of Assay using Bacillus cereus spores

### 1) Plate format

A sample (50µl) comprising a suspension of B.cereus spores (1 x 10⁸ per ml) in distilled sterile water was added to each well of a blocked ELISA plate (Immulon microtitre ELISA plate) which was then placed in an oven at 37°C overnight to dry the spores onto the plates.

The plates were then washed three times with a wash solution comprising 0.05% v/v Tween 20 in phosphate buffered saline (PBS) or PBST.

A blocking buffer (200ml) comprising 2% w/v dry milk powder and 0.05% v/v Tween 20 PBS was added to each well. The plate was then sealed and incubated at room temperature for a minimum of 1 hour. It was then washed again three times in PBST.

A solution of primary antibody expressing M13 phage (1x 10⁹ transforming units per ml), wherein the primary antibody is a B.cereus specific single chain variable fragment (scFv), in PBST blocking buffer was prepared and at least 50µl added per well. PBST blocking buffer was added to one of the wells in place of the primary antibody expressing phage as a negative control.

The plate was incubated at 37°C for 1 hour, then washed 5 times in PBST. After drying, 50 µl dH₂O was added to each well. The plate was then boiled for 30 seconds to free the phage for PCR. After allowing the plate to cool briefly, and contents of the wells were transferred to a PCR tube, together with a conventional PCR mix (18 µl) including M13 phage specific primers and SybrGreen, used in accordance with the manufacturer's instructions.

The sample subjected to a series of thermal cycling steps on the Roche LightCycler as follows:
94°C for 0 seconds (melt);
62°C for 30 seconds (annealing phase);
72°C for 30 seconds (extension phase).

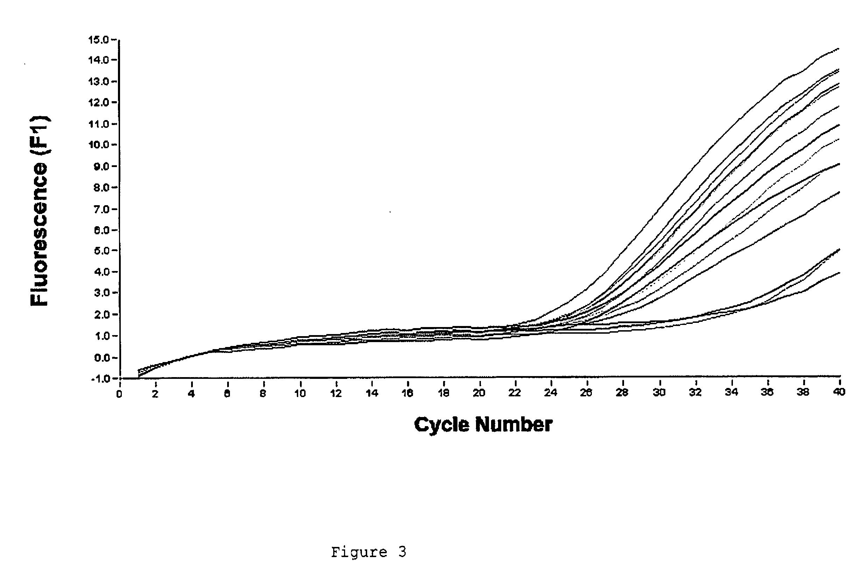

40 cycles were carried out. The fluorescent signal from the samples was monitored once per cycle at the end of the extension phase. The process was repeated with an increasingly dilute sample and the results are shown in Figure 3.

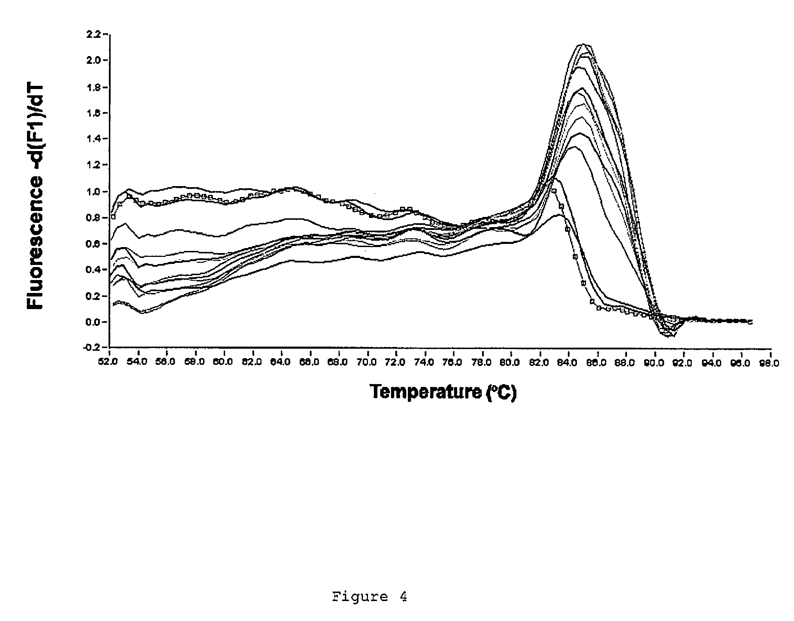

Negative control samples showed only a small increase in signal at the end of the cycling process. It was confirmed by a final melt curve analysis (Figure 4) that the signal from the negative control was due to non-specific products such as primer-dimers.

The results show however that the presence of bacterial spores in the samples was detectable using this method.

### Example 2

### Detection Assay

For use as a detection assay, a sample is added to a blocked-antibody coated ELISA plate and incubated at 37°C for 5 to 60 minutes.

Thereafter, the plate is washed with wash liquid from three to five times.

A suspension of filamentous phage expressing an scFv specific for the assay target is added to the plate, and incubated for 5-60 mins. After further washing (3-5 times), conventional PCR reagents are added, together with a suitable reporter system such as the SybrGreen dye mentioned above. However, other reporter mechanisms, for example using fluorescent reporter probes, such as TASMAN® or other probes for in situ monitoring may be employed. The reaction mixture is thermally cycled to effect the amplification in the conventional way.

The PCR cycle number at which product appears (fluorescence threshold crossing point) is noted and correlated with the concentration of analyte in original sample.

It is possible to add more than one scFv with different specificities at the same time, to determine a range of targets. In such cases, melt profiles may be carried out to distinguish which one of the scFv is present and therefore has bound to the analyte. If desired, phage sequences or antibiotic resistance sequences found in the transformed phage may be used as an internal reference for the PCR.

### Example 3

### Alternative Filtration Assay Format (example of the invention claimed)

In this embodiment, a liquid sample is passed through a 0.2 or 0.45 micron filter, depending upon the nature of the assay target, and the filter is then washed. Target analyte, for example bacterial spores, are retained on the filter. Subsequently, a suspension of filamentous phage expressing an scFv specific for the assay target is also passed through the filter, which is again washed. Any phage which binds to the target on the filter can then be detected, by PCR as described above.

### Example 4

### Detection of phage displaying single chain antibodies directed against B cereus in an immunoassay format.

50µl of 10⁷ *B. cereus* spores/ml were diluted 10 fold in dH₂O down an Immulon 2 ELISA plate spores and dried onto the plate at 37°C overnight. This immobilised the spores on the plate so that they mirrored the situation in which an analyte was binding an immobilised antibody, as might occur in an assay for the spores.

The plate was then washed in dH₂-O, three times. Each well was then blocked by the addition of 150µl of 1% blotto/phosphate buffered saline (PBS) and the plate was then incubated at 37 °C for 1 hour. The plate was washed in PBS-Tween, three times. 50µl of phage suspension in 1% blotto/PBS was added to each well and then the plate was incubated at 37°C for one hour, so that the phage bound to the spores on the plate. The plate was then washed in PBS-Tween 3 times, followed by three washes in dH₂O to remove unbound phage.

50µl of dH₂O was then added to each well and the plate was heated in boiling water for 30 seconds so as to elute the phage.

2µl from each well were then assayed by PCR using phage directed primers, amplify the lacI gene found within the M13 derived phage. Real-time PCR was performed using a Corbett RotorGene. Each tris-buffered reaction contained 0.5µM each of forward and reverse LacI primers, 0.3 µM of lacI specific TaqMan probe, 4 mM MgCl₂. Cycling parameters were 95° for 5 seconds and 60°C for 1 minute for 50 cycles. The primers, probe and target were as follows:

| | |
|---|---|
| *FORWARD PRIMER* | *5'-CGTGGTGGTGTCGATGGTAG* |
| *REVERSE PRIMER* | *5'-TGTGCACCGCTTT* |
| *PROBE SEQUENCE* | *5'- ACGAAG CGGCGTCGAA GCCTG* |
| AMPLICON | 5′-CGTGGTGGTGTCGATGGTAGAACGA AGCGGC GTCGAAGCCTGTAAGCGGCGGTGCACA |

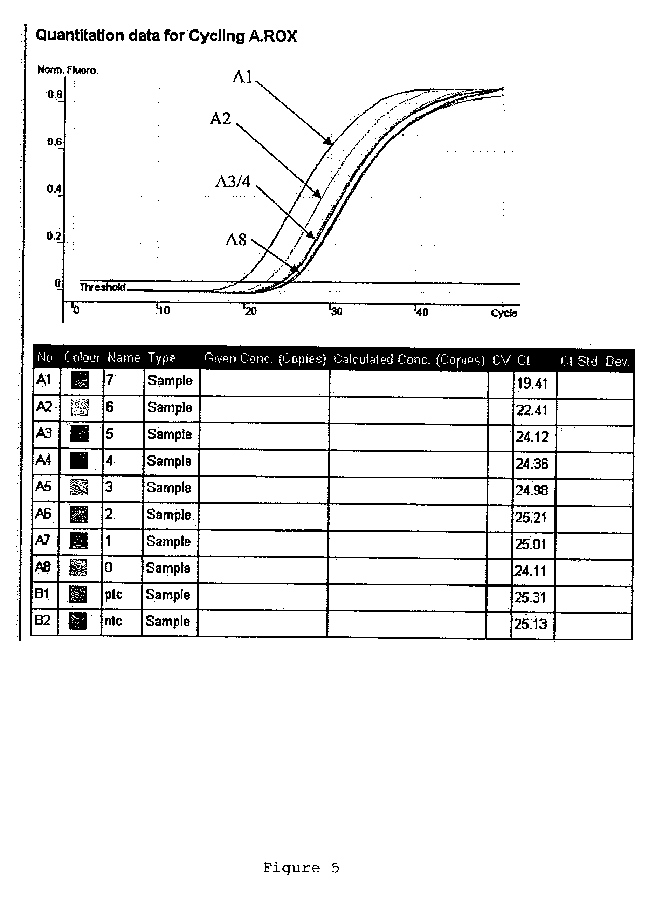

The results are shown in Figure 5. The results show that 10⁶ to 10³ spores per well were detectable above background level, even though the background level in this case was quite high. This was probably as a result of cross-contamination. There are shared genes between M13 derived phages and M13 derived cloning vectors used routinely in the lab. The sensitivity of the assay could be readily improved by setting up the phage PCR reaction in a lab that is free of M13 contamination or by choosing primers specific to phages displaying *B. cereus* antibodies.

## Claims

1. A method of detecting an analyte in a liquid sample, said method comprising passing said sample through a filter so that analyte is retained on the filter, washing the filter, passing a suspension of filamentous phage expressing an scFV specific for an assay target selected from the analyte or an analogue thereof, or a binding moiety for the analyte, through the filter, washing the filter, detecting any phage which has bound to the assay target on the filter by detecting the presence or absence of phage DNA on the filter using a polymerase chain reaction, and relating that to the presence or absence of analyte in the sample.

2. A method according to claim 1 wherein the filter is a 0.2 micron filter.

3. A method according to claim 1 wherein the filter is a 0.45 micron filter.

4. A method according to any one of the preceding claims wherein the analyte comprises bacterial spores.

5. A method according to any one of claims 1 to 4 wherein the suspension of filamentous phage comprises a multi-specificity mixture of viruses.

6. A method according to claim 5 wherein detection of multiple nucleic acid sequences, each characteristic of individual viruses, is carried out to detect the presence or absence of phage.

7. A method according to any preceding claim wherein the amplification reaction is carried out in the presence of a DNA binding reagent, or a primer or probe which is labelled with a fluorescent label.

8. A method according to any one of claims 1 to 7 wherein the amount of filamentous phage detected is quantified, and this is related to the amount of analyte in the sample.

9. A method according to any one of claims 1 to 8 wherein phage is detected by detecting a nucleic acid sequence which is characteristic of a particular phage used in the method.

10. A method according to claim 9 wherein the phage is a recombinant virus which has been transformed with a marker sequence, and this sequence is the sequence which is detected.

11. A method according to claim 9 wherein sequences characteristic of more than one virus are detected.

## Patentansprüche

1. Verfahren zum Nachweis eines Analyten in einer flüssigen Probe, wobei das Verfahren umfasst das Passieren der Probe durch ein Filter, so dass der Analyt auf dem Filter zurückgehalten wird, das Waschen des Filters, das Passieren einer Suspension von filamentösem Phagen, der ein scFv, welches für ein Assaytarget, ausgewählt aus dem Analyten oder einem Analogon davon, spezifisch ist, oder eine Bindungsgruppe für den Analyten exprimiert, durch das Filter, Waschen des Filters, Detektieren von Phagen, der an das Assaytarget auf dem Filter gebunden hat, durch Nachweis der Gegenwart oder Abwesenheit von Phagen-DNA auf dem Filter unter Anwendung einer Polymerase-Kettenreaktion und Inbeziehungsetzen desselben mit der Gegenwart oder Abwesenheit von Analyt in der Probe.

2. Verfahren nach Anspruch 1, worin das Filter ein 0,2 Mikron Filter ist.

3. Verfahren nach Anspruch 1, worin das Filter ein 0,45 Mikron Filter ist.

4. Verfahren nach irgendeinem vorangehenden Anspruch, worin der Analyt bakterielle Sporen umfasst.

5. Verfahren nach irgendeinem der Ansprüche 1 bis 4, worin die Suspension von filamentösem Phagen eine multi-spezifische Virusmischung umfasst.

6. Verfahren nach Anspruch 5, worin eine Detektion von multiplen Nucleinsäuresequenzen, jede charakteristisch für individuelle Viren, durchgeführt wird, um die Gegenwart oder Abwesenheit von Phagen nachzuweisen.

7. Verfahren nach irgendeinem vorangehenden Anspruch, worin die Amplifikationsreaktion in Gegenwart eines DNA-Bindungsreagens oder eines Primers oder einer Sonde mit einem Fluoreszenzmarker durchgeführt wird.

8. Verfahren nach irgendeinem der Ansprüche 1 bis 7, worin die nachgewiesene Menge an filamentösem Phagen quantifiziert wird und dies mit der Menge an Analyt in der Probe in Beziehung gesetzt wird.

9. Verfahren nach irgendeinem der Ansprüche 1 bis 8, worin der Phagen nachgewiesen wird durch Detektion einer Nucleinsäuresequenz, die für einen bestimmten in dem Verfahren verwendeten Phagen charakteristisch ist.

10. Verfahren nach Anspruch 9, worin der Phagen ein rekombinantes Virus ist, das mit einer Markersequenz transformiert worden ist, und diese Sequenz die nachgewiesene Sequenz ist.

11. Verfahren nach Anspruch 9, worin Sequenzen, die für mehr als ein Virus charakteristisch sind, nachgewiesen werden.

## Revendications

1. Méthode pour détecter un analyte dans un échantillon liquide, ladite méthode comprenant : faire passer ledit échantillon à travers un filtre de manière à ce que l'analyte soit retenu sur le filtre, laver le filtre, faire passer une suspension de phage filamenteux exprimant un scFV spécifique d'une cible testée choisie parmi l'analyte ou un analogue de celui-ci, ou un fragment de liaison à l'analyte, à travers le filtre, laver le filtre, détecter tout phage qui s'est lié à la cible testée sur le filtre en détectant la présence ou l'absence d'ADN phagique sur le filtre au moyen d'une amplification en chaîne par polymérase, et associer cela à la présence ou à l'absence d'analyte dans l'échantillon.

2. Méthode selon la revendication 1, dans laquelle le filtre est un filtre de 0,2 micron.

3. Méthode selon la revendication 1, dans laquelle le filtre est un filtre de 0,45 micron.

4. Méthode selon l'une quelconque des revendications précédentes, dans laquelle l'analyte comprend des spores bactériens.

5. Méthode selon l'une quelconque des revendications 1 à 4, dans laquelle la suspension de phage filamenteux comprend un mélange multispécifique de virus.

6. Méthode selon la revendication 5, dans laquelle la détection de plusieurs séquences d'acides nucléiques, chacune étant caractéristique de virus individuels, est effectuée pour détecter la présence ou l'absence de phage.

7. Méthode selon l'une quelconque des revendications précédentes, dans laquelle la réaction d'amplification est réalisée en présence d'un réactif de liaison à l'ADN, ou bien d'une amorce ou d'une sonde marquée avec un marqueur fluorescent.

8. Méthode selon l'une quelconque des revendications 1 à 7, dans laquelle la quantité de phage filamenteux détecté est quantifiée et ceci est associé à la quantité d'analyte dans l'échantillon.

9. Méthode selon l'une quelconque des revendications 1 à 8, dans laquelle le phage est détecté en détectant une séquence d'acides nucléiques qui est caractéristique d'un phage particulier employé dans la méthode.

10. Méthode selon la revendication 9, dans laquelle le phage est un virus recombinant qui a été transformé avec une séquence marqueur et cette séquence est la séquence qui est détectée.

11. Méthode selon la revendication 9, dans laquelle on détecte les séquences caractéristiques de plus d'un virus.


### SEQUENCE LISTING

<110> The Secretary of State for Defence
<120> Use of a Virus Expressing a Binding Moiety to Measure Analytes in a sample
<130> RT/P1076EP02
<150> EP 07011811.2
   <151> 2004-03-02
<150> EP 04716274.8
   <151> 2004-03-02
<150> PCT/GB2004/000865
   <151> 2004-03-02
<150> GB 0304832.9
   <151> 2003-03-04
<160> 4
<170> PatentIn version 3.3
<210> 1
   <211> 20
   <212> DNA
   <213> artificial sequence
<220>
   <221> source
   <222> 1..20
   <223> /note= "Description of artificial sequence: Forward primer"
<400> 1
   cgtggtggtg tcgatggtag 20
<210> 2
   <211> 13
   <212> DNA
   <213> artificial sequence
<220>
   <221> source
   <222> 1..13
   <223> /note= "Description of artificial sequence: Reverse primer"
<400> 2
   tgtgcaccgc ttt 13
<210> 3
   <211> 21
   <212> DNA
   <213> artificial sequence
<220>
   <221> source
   <222> 1..21
   <223> /note= "Description of artificial sequence: Probe"
<400> 3
   acgaagcggc gtcgaagcct g 21
<210> 4
   <211> 58
   <212> DNA
   <213> artificial sequence
<220>
   <221> source
   <222> 1..58
   <223> /note= "Description of artificial sequence: Amplicon"
<400> 4
   cgtggtggtg tcgatggtag aacgaagcgg cgtcgaagcc tgtaagcggc ggtgcaca 58